(19) Europäisches Patentamt
European Patent Office
Office européen des brevets

(11) Publication number: **0 276 552 A1**

# (12) EUROPEAN PATENT APPLICATION

(21) Application number: **87310787.4**

(51) Int. Cl.⁴: **C07D 211/90**, A61K 31/44

(22) Date of filing: **08.12.87**

(30) Priority: **08.12.86 JP 292694/86**

(43) Date of publication of application: **03.08.88 Bulletin 88/31**

(84) Designated Contracting States: **AT BE CH DE ES FR GB IT LI NL SE**

(71) Applicant: **YAMANOUCHI PHARMACEUTICAL CO. LTD.**
**No. 3-11 Nihonbashi-Honcho, 2-chome**
**Chuo-ku**
**Tokyo(JP)**

(72) Inventor: **Fujikura, Takashi**
**3-17, Shirahata 4-chome**
**Urawa-shi Saitama(JP)**
Inventor: **Matsumoto, Yuzo**
**16-1, Hasune 3-chome**
**Itabashi-ku Tokyo(JP)**
Inventor: **Hara, Ryuichiro**
**3-19-11, Honcho**
**Nakano-ku Tokyo(JP)**
Inventor: **Asano, Masaharu**
**1-10-A206, Aioi-cho**
**Itabashi-ku Tokyo(JP)**

(74) Representative: **Geering, Keith Edwin et al**
**REDDIE & GROSE 16 Theobalds Road**
**London WC1X 8PL(GB)**

## (54) 1,4-dihydropyridine derivatives and their production.

(57) 1,4-dihydropyridine derivatives of formula (I) below, and their salts

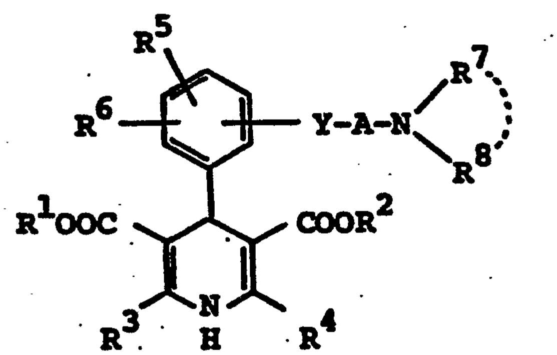

(I)

wherein
$R^1$ and $R^2$ are the same or different and selected from alkyl groups which may be interrupted at least once by an oxygen atom, cycloalkyl-substituted $C_1$-$C_6$ alkyl groups and halogen-substituted $C_1$-$C_6$ alkyl groups;
$R^3$ and $R^4$ are the same or different and selected from $C_1$-$C_6$ alkyl groups;
$R^5$ and $R^6$ are the same or different and selected from hydrogen and halogen atoms and nitro, $C_1$-$C_6$ alkyl,

EP 0 276 552 A1

$C_1$-$C_6$ alkoxy, $C_1$-$C_6$ alkylthio, $C_1$-$C_6$ alkylsulfonyl and $C_1$-$C_6$ alkylsulfinyl groups;
$R^7$ is a hydrogen atom or $C_1$-$C_6$ alkyl group and
$R^8$ is a $C_1$-$C_6$ alkyl, hydroxy-($C_1$-$C_6$) alkyl, aralkyl, aryl, $C_1$-$C_6$ acyl, $C_1$-$C_6$ alkylsulfonyl, aryloxy-$C_1$-$C_6$ alkyl or aryloxy-$C_1$-$C_6$ alkoxy group or $R^7$ and $R^8$ form together with the amino nitrogen a pyrrole, piperidine, morpholine, piperazine or azepine ring which may be substituted by $C_1$-$C_6$ alkyl or aralkyl;
A is an alkylene, alkenylene or alkynylene group; and
Y is an oxygen or sulfur atom are medically useful as calcium antagonists of long lasting activity.

## 1,4-DIHYDROPYRIDINE DERIVATIVES AND THEIR PRODUCTION

The present invention relates to 1,4-dihydropyridine derivatives of formula (I) below, and their salts, which are useful as medical drugs (especially as durable calcium antagonists), and to processes for their production:

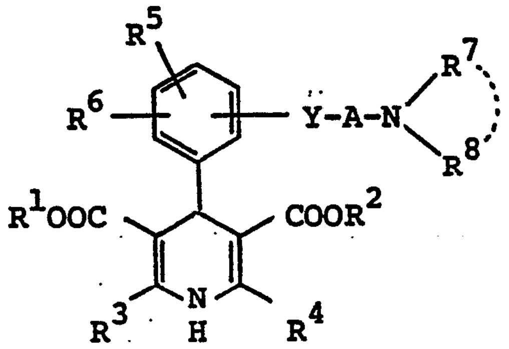

(I)

wherein
$R^1$ and $R^2$ are the same or different and selected from alkyl groups which may be interrupted at least once by an oxygen atom, cycloalkyl-substituted $C_1$-$C_6$ alkyl groups and halogen-substituted $C_1$-$C_6$ alkyl groups;
$R^3$ and $R^4$ are the same or different and selected from $C_1$-$C_6$ alkyl groups;
$R^5$ and $R^6$ are the same or different and selected from hydrogen and halogen atoms and nitro, $C_1$-$C_6$ alkyl, $C_1$-$C_6$ alkoxy, $C_1$-$C_6$ alkylthio, $C_1$-$C_6$ alkylsulfonyl and $C_1$-$C_6$ alkylsulfinyl groups;
$R^7$ is a hydrogen atom or $C_1$-$C_6$ alkyl group and
$R^8$ is a $C_1$-$C_6$ alkyl, hydroxy-($C_1$-$C_6$) alkyl, aralkyl, aryl, $C_1$-$C_6$ acyl, $C_1$-$C_6$ alkylsulfonyl, aryloxy-$C_1$-$C_6$ alkyl or aryloxy-$C_1$-$C_6$ alkoxy group or $R^7$ and $R^8$ form together with the amino nitrogen a pyrrole, piperidine, morpholine, piperazine or azepine ring which may be substituted by $C_1$-$C_6$ alkyl or aralkyl;
A is an alkylene, alkenylene or alkynylene group; and
Y is an oxygen or sulfur atom.

Since it was confirmed that Nifedipine or Nicardipine exhibits cerebro-and/or coronary vasodilating action based on calcium antagonistic activity, a variety of calcium antagonists containing the 1,4-dihydropyridine skeleton have been developed.

The compounds of the present invention are novel compounds characterized structurally in that substituents on the 4-phenyl group are different from those of known 1,4-position pyridine calcium antagonists and pharmacologically in that the durability of their calcium antagonistic activity is superior to that of known calcium antagonists.

It is disclosed in European Patent Nos. 167,371 and 194,751 that compounds represented by general formula:

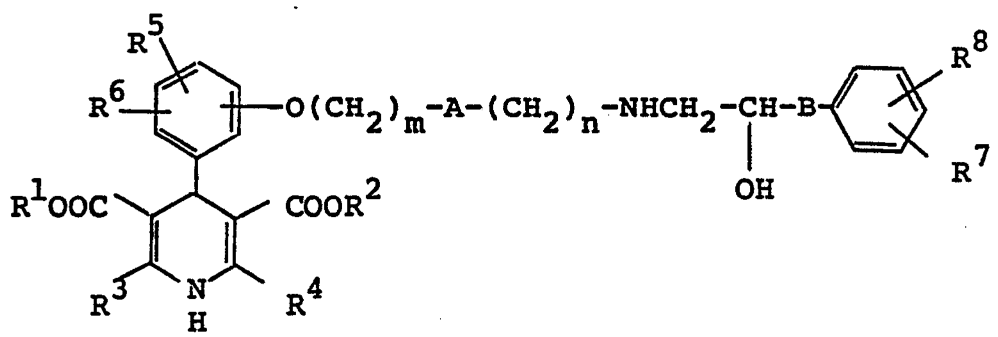

( see No.167,371 for identity of the substituents)

have both calcium receptor antagonistic activity and β-blocking activity. The compounds of the present invention lack β-blocking activity and exhibit calcium receptor antagonistic activity alone, this activity being longer lasting than that of conventional compounds.

It is disclosed in Published Unexamined Japanese Patent Application No. 136558/1985 that compounds represented by general formula:

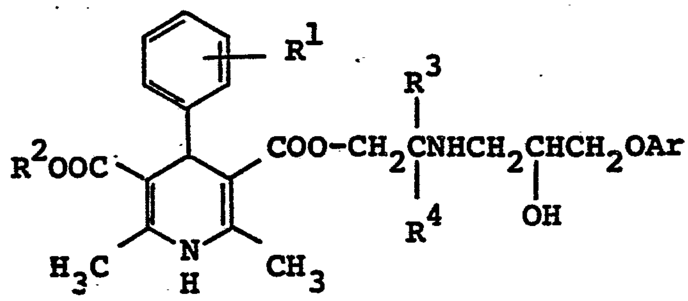

(see the published specification for the identity of the substituents)

have both calcium receptor antagonistic activity and β-blocking activity. The compounds of the present invention are novel and differ not only as aforesaid (pharmacologically) but also in their structure.

In this specification, the term "lower" refers to a straight or branched carbon chain having up to 6 carbon atoms, unless otherwise indicated. Accordingly, concrete examples of the lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl groups, etc.

Further as the "lower alkoxy group", mention may be made of methoxy, ethoxy, propoxy, isopropoxy, butoxy, isobutoxy, sec-butoxy, tert-butoxy, pentyloxy (amyloxy), isoptentyloxy, tert-pentyloxy, neopentyloxy, 2-methylbutoxy, 1,2-dimethylpropoxy, 1-ethylpropoxy and hexyloxy groups and the like.

An "aryloxy-lower alkoxy group" is one obtainable by substituting any hydrogen atom in a "lower alkoxy group" with an "aryloxy group" and concrete examples of the "aryloxy group" include phenoxy and napthyl groups and the like, with particular preference for phenoxy. Accordingly, exemplifying specific "aryloxy-lower alkyl group" by the phenoxy type, mention may be made of phenoxymethyl, phenoxyethyl, phenoxypropyl, 1-methyl-2-phenoxyethyl, phenoxybutyl, 1-methyl-3-phenoxypropyl, phenoxypentyl and phenoxyhexyl groups and the like.

A "lower alkylthio group" is a "lower alkoxy group" with the oxygen replaced by a sulfur atom and specific examples include methylthio, ethylthio, propylthio, isopropylthio, butylthio, sec-butylthio, tert-butylthio, pentylthio, neopentylthio, 2-methylbutylthio, 1,2-dimethylpropylthio, 1-ethylpropylthio and hexylthio groups and the like.

A "lower alkylsulfonyl" or "lower alkylsulfinyl" group is a sulfonyl or sulfinyl group substituted with a "lower alkyl group"; specific examples are methylsulfonyl (or sulfinyl), ethylsulfonyl (or sulfinyl), propylsulfonyl (or sulfinyl), butylsulfonyl (or sulfinyl), isobutylsulfonyl (or sulfinyl), pentylsulfonyl (or sulfinyl), neopentylsulfonyl (or sulfinyl) and hexylsulfonyl (or sulfinyl) groups and the like.

A "cycloalkyl-substituted lower alkyl group" is one obtainable by substituting any hydrogen atom in a "lower alkyl group" with a "cycloalkyl group" and specific examples fo the "cycloalkyl group" are preferably alicyclic groups having 3 to 6 carbon atoms such as cyclopropyl, cyclobutyl, cyclopentyl and cyclohexyl groups, etc. Accordingly, concrete examples of the "cycloalkyl-substituted lower alkyl group" are cyclohexylmethyl, 2-cyclohexylethyl, 3-cyclohexylpropyl, 2-cyclohexyl-1-methylethyl, 4-cyclohexylbutyl, 5-cyclohexylpentyl and 6-cyclohexylhexyl groups, etc. (when illustrated by cyclohexyl groups).

A "halogen-substituted lower alkyl group" is one obtainable by substituting any 1 to 3 hydrogen atoms

in a "lower alkyl group" with halogen(s) preferably selected from fluorine, chlorine and bromine atoms. Accordingly, in terms of chlorine substitution, concrete examples of the "halogen-substituted lower alkyl group" are chloromethyl, trichloromethyl, 2-chloroethyl, 3-chloropropyl, 2-chloro-1-methylethyl, 4-chlorobutyl, 5-chloropentyl and 6-chlorohexyl groups and the like.

A "hydroxy-lower alkyl group" is one obtainable by substituting any hydrogen atom in a "lower alkyl group" with a hydroxy group and concrete examples are hydroxymethyl, 2-hydroxyethyl, 1-hydroxyethyl, 3-hydroxypropyl, 2-hydroxypropyl, 2-hydroxy-2-methylethyl, 1-hydroxy-2-methylethyl, 4-hydroxybutyl, 5-hydroxypentyl and 6-hydroxyhexyl groups and the like.

An "aryloxy-lower alkyl group" is one obtainable by substituting any hydrogen atom in a "lower alkyl group" with an "aryloxy group" and as "aryloxy groups there are eg. phenoxy and naphthyloxy groups, etc., with phenoxy being particularly preferred. Accordingly, concrete examples of the "aryloxy-lower alkyl group" are (in terms of phenoxy group) phenoxymethyl, phenoxyethyl, phenoxypropyl, 1-methyl-2-phenoxyethyl, phenoxybutyl, 1-methyl-3-phenoxypropyl, phenoxypentyl and phenoxyhexyl and the like.

An "alkyl group which may be interrupted at least once by an oxygen atom"can be an "alkyl group" or an "alkyl group interrupted once or more by an oxygen atom". The preferred "alkyl groups", in each case are straight or branched groups having 1 to 10 carbon atoms. Accordingly, in addition to the concrete examples of the aforesaid "lower alkyl group", concrete examples of $R^1$ and $R^2$ include similar $C_7$ to $C_{10}$ alkyl groups and methoxymethyl, 1-methoxyethyl, 2-methoxyethyl, ethoxyethyl, 3-methoxypropyl, 2-methoxypropyl, 2-ethoxyethyl, methoxybutyl, methoxypentyl, ethoxymethyl, ethoxyethyl, propoxymethyl, isopropoxymethyl, 4-methoxybutyl, 3-ethoxypropyl, 2-propoxyethyl, butoxymethyl, 5-methoxypentyl, 4-ethoxybutyl, 2-(2-ethoxyethoxy)ethyl, [3,6-dioxaoctyl], 2-butoxyethyl, 2-pentyloxymethyl, 6-methoxyhexyl, 5-ethoxypentyl, 2-pentyloxyethyl, hexyloxymethyl, 7-methoxyheptyl, 6-ethoxyhexyl, 2-[2-(2-ethoxyethoxy)ethoxy]ethyl, [3,6,9-trioxaundecyl], 2-hexyloxyethyl, heptyloxymethyl, 8-methoxyoctyl, 7-ethoxyheptyl, 2-heptyloxyethyl, octyloxymethyl and 9-methoxynonyl groups and the like.

As an "aryl group", there are as concrete examples phenyl and naphthyl groups and the like but particularly preferred is phenyl.

An "aralkyl group" is one obtainable by substituting any hydrogen atom in a "lower alkyl group" with an "aryl group" - e.g. a benzyl, phenethyl or phenylpropyl group, etc.

An alkylene group A preferably has 1 to 12 carbon atoms. Specific examples are methylene, ethylene,

methylmethylene 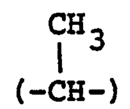,

trimethylene,

propylene 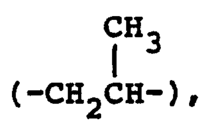, 2-propylene 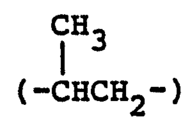,

dimethylmethylene 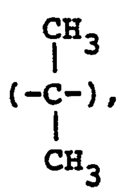, tetramethylene,

1-methyltrimethylene, 2-methyltrimethylene, 3-methyltrimethylene, 1-ethylethylene,

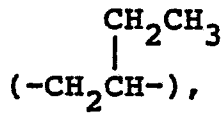, 2-ethylethylene 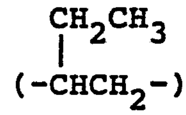, 2,2-dimethylethylene 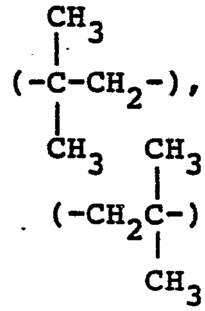, 1,1-dimethylethylene, ethylmethylmethylene 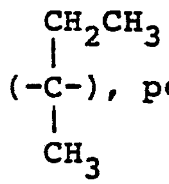, pentamethylene, 2-methyltetramethylene, 3-methyltetramethylene, 4-methyltetramethylene, 1,1-dimethyltrimethylene, 2,2-dimethyltrimethylene, 3,3-dimethyltrimethylene, 1,3-dimethyltrimethylene, 2,3-dimethyltrimethylene, 1,2-dimethyltrimethylene, 1-ethyltrimethylene, 1,1,2-trimethylethylene, diethylmethylene, hexamethylene, 1-methylpentamethylene, 1,1-dimethyltetramethylene, 2,2-dimethyl tetramethylene, 3,3-dimethyltetramethylene, 4,4-dimethyltetramethylene, 1,1,3-trimethyltrimethylene, 1,1,2-trimethyltrimethylene, 1,1,2,2-tetramethylethylene, 1,1-dimethyl-2-ethylethylene, 1,1-diethylethylene, heptamethylene, 1-methylhexamethylene, 1,1-dimethylpentamethylene, 2,2-dimethylpentamethylene, 3,3-dimethylpentamethylene, 4,4-dimethylpentamethylene, 5,5-dimethylpentamethylene, 1,1,4-trimethyltetramethylene, 1,1,2-trimethyltetramethylene, 1,1,3-trimethyltetramethylene, 1,1,2,2-tetramethyltrimethylene, 1,1,3,3-tetramethyltrimethylene, 1,1-dimethyl-2-ethyltrimethylene, 1,1-dimethyl-3-ethyltrimethylene, octamethylene, 1-methylheptamethylene, 1,1-dimethylhexamethylene, nonamethylene, 1-methyloctamethylene, 1,1-dimethylheptamethylene, decamethylene, 1-methylnonamethylene, 1,1-dimethyloctamethylene, undecamethylene, 1-methyldecamethylene, 1,1-dimethylnonamethylene, dodecamethylene and 1,1-dimethyldecamethylene groups etc.

An alkenylene group A preferably has 2 to 12 carbon atoms; concrete examples are vinylene, propenylene, ($-CH_2CH=CH-$), 2-propenylene ($-CH=CH-CH_2-$), 1-methylvinylene, 2-methylvinylene, butenylene, 2-butenylene, 3-butenylene, 1-methylpropenylene, 1-methyl-2-propenylene, pentenylene, 1-methylbutenylene, 1-methyl-2-butenylene, 1-methyl-3-butenylene, 1,1-dimethyl-2-propenylene, hexenylene, 2-hexenylene, 3-hexenylene, 4-hexenylene, 5-hexenylene, 1-methyl-2-pentenylene, 1-methyl-3-pentenylene, 1,1-dimethyl-2-butenylene, 1,1-dimethyl-3-butenylene, heptenylene, 2-heptenylene, 3-heptenylene, 4-heptenylene, 5-heptenylene, 6-heptenylene, 1,1-dimethyl-2-pentenylene, 1,1-dimethyl-3-pentenylene, 1,1-dimethyl-4-pentenylene, 2-octenylene, 4-octenylene, 7-octenylene, 1,1-dimethyl-2-hexenylene, 1,1-dimethyl-3-hexenylene, 1,1-dimethyl-5-hexenylene, 2-nonenylene; 4-nonenylene, 5-nonenylene, 8-nonenylene, 1,1-dimethyl-2-heptenylene, 1,1-dimethyl-3-heptenylene, 1,1-dimethyl-4-heptenylene, 1,1-dimethyl-6-heptenylene, 2-decenylene, 5-decenylene, 9-decenylene, 1,1-dimethyl-4-octenylene, 1,1-dimethyl-7-octenylene, 2-undecenylene, 5-undecenylene, 6-undecenylene, 10-undecenylene, 1,1-diemthyl-2-nonenylene, 1,1-dimethyl-4-nonenylene, 1,1-dimethyl-5-nonenylene, 1,1-dimethyl-8-nonenylene, 2-dodecenylene, 6-dodecenylene, 11-dodecenylene, 1,1-dimethyl-2-dodecenylene, 1,1-dimethyl-5-dodecenylene and 1,1-dimethyl-9-dodecenylene groups etc.

An alkynylene group A preferably has 2 to 12 carbon atoms and examples are ethynylene, propynylene ($-CH_2-C\equiv C-$), 2-propynylene ($-C\equiv C-CH_2-$), butynylene, 2-butynylene, 3-butynylene, 1-methyl-2-propynylene, pentynylene, 2-pentynylene, 3-pentynylene, 4-pentynylene,

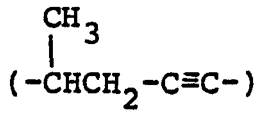, 1-methyl-2-butynylene,

4-methyl-2-butynylene, 1,1-dimethyl-2-propynylene 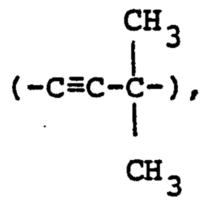, hexynylene,

2-hexynylene, 3-hexynylene, 4-hexynylene, 5-hexynylene, 1-methyl-3-pentynylene, 1-methyl-4-pentynylene, 1,1-dimethyl-2-butynylene, 1,1-dimethyl-3-butynylene, heptynylene, 2-heptynylene, 3-heptynylene, 4-heptynylene, 5-heptynylene, 6-heptynylene, 1,1-dimethyl-2-pentynylene, 1,1-dimethyl-4-pentynylene, 2-octynylene, 1,1-dimethyl-2-hexynylene, 1,1-dimethyl-3-hexynylene, 1,1-dimethyl-5-hexynylene, 4-nonynylene, 5-nonynylene, 8-nonynylene, 1,1-dimethyl-2-heptynylene, 1,1-dimethyl-3-heptynylene, 1,1-dimethyl-4-heptynylene, 1,1-dimethyl-6-heptynylene, 2-decynylene, 5-decynylene, 9-decynylene, 1,1-dimethyl-2-octynylene, 1,1-dimethyl-4-octynylene, 1,1-dimethyl-7-octynylene, 2-undecynylene, 5-undecynylene, 6-undecynylene, 10-undecynylene, 1,1-dimethyl-2-nonynylene, 1,1-dimethyl-4-nonynylene, 1,1-dimethyl-5-nonynylene, 1,1-dimethyl-8-nonynylene, 2-dodecynylene, 6-dodecynylene, 11-dodecynylene, 1,1-dimethyl-2-dodecynylene, 1,1-dimethyl-5-dodecynylene and 1,1-dimethyl-9-dodecynylene groups etc.

The present invention includes the salts of compounds (I), and examples of such salts include acid addition salts with inorganic acids such as mineral acids (e.g. hydrochloric, hydrobromic, hydroiodic, sulfuric, nitric and phosphoric acids and the like) and with various organic acids such as formic, acetic, oxalic, citric, succinic, fumaric, maleic, malic, tartaric, methanesulfonic and ethanesulfonic acids and the like.

Some compounds according to the invention can evidently have various isomers such as optical isomers, diastereoisomers and geometrical isomers. The present invention includes isolated isomers and any mixtures thereof.

The present invention also includes processes of producing the compounds represented by general formula (I). The compounds (I) of the present invention can be prepared by various processes. Representative examples of the processes will be given below.

**Process 1:**

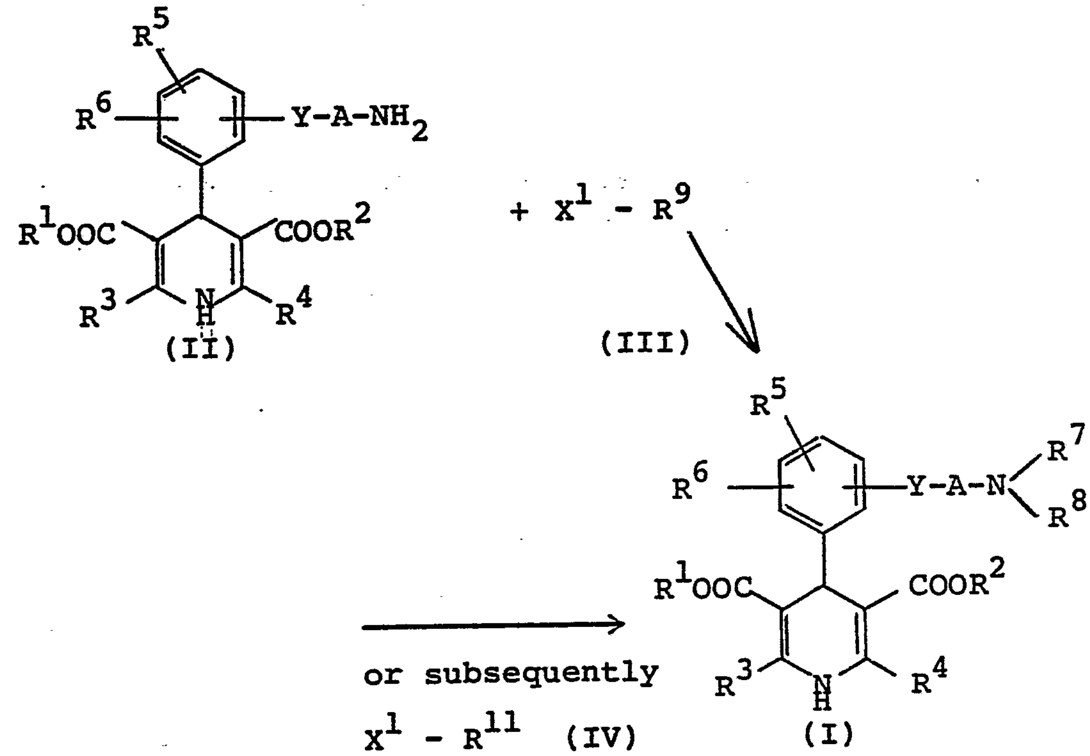


wherein $R^1$, $R^2$, $R^3$, $R^4$, $R^5$, $R^6$, $R^7$, $R^8$, Y and A are as defined above; $R^9$ is lower alkyl, hydroxy-lower alkyl, aralkyl, aryl, lower acyl, lower alkylsulfonyl, aryloxy-lower alkyl or aryloxy-lower alkoxy or a group - B - $X^2$; $X^1$ is a halogen atom, an organic sulfonic acid residue or a lower acyloxy group; B is tetramethylene, pentamethylene, hexamethylene, 3-oxapentamethylene, ($-CH_2CH_2OCH_2CH_2-$) or a group :

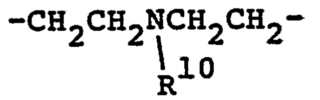 ;

$X^2$ is a halogen atom(the same as or different from $X^1$ when a halogen atom); $R^{10}$ is a hydrogen atom or lower alkyl or aralkyl; and $R^{11}$ is a lower alkyl.

Herein as the halogen atoms mention may be made of iodine, bromine, chlorine, etc.

Examples of the organic sulfonic acid residue are alkanesulfonic acid residues such as methanesulfonyloxy and ethanesulfonyloxy groups and the like, and aromatic sulfonic acid residues such as benzenesulfonyloxy and toluene (in particular, p-toluene)sulfonyloxy groups and the like. As the lower acyloxy group, mention may be made of acetoxy, propionyloxy, etc.

Thus compounds of the present invention can be produced by:

(1) reacting amines (II) with halides, acid anhydrides or sulfonates (III); or

(2) reacting halides, acid anhydrides or sulfonates (IV) with secondary amines obtained in (1).

When the compound (III) is - $X^1$ -B - $X^2$, $X^1$ and $X^2$ are both halogen atoms, preferably the same halogen atom.

The reactions (1) and (2) can be carried out under similar reaction conditions and will be described in detail, dividing into cases wherein $X^1$ represents (a) a halogen atom or lower acyloxy group and (b) an organic sulfonic acid residue.

The reactions (a) can proceed in the absence of any solvent but it is generally advantageous to conduct the reaction in organic solvents such as benzene, toluene, xylene, dimethylformamide, dimethylsulfoxide, acetonitrile, dichloromethane, dichloroethane, etc.

Compounds (II) and (III) can be used in almost equimolar amounts or with compounds (III) in slight excess.

The reaction is preferably conducted at room temperature or with heating or with heating under reflux.

For smooth reaction it is sometimes advantageous to add secondary or tertiary bases such as pyridine, picoline, N,N-dimethylaniline, N-methylmorpholine, trimethylamine, triethylamine, dimethylamine, or the like; or inorganic bases such as potassium carbonate, sodium carbonate, sodium hydrogencarbonate, or the like.

To reduce side reaction, it is possible to protect the amino group of the compounds (II), splitting the protective group off after the reaction. Such protective groups include toluenesulfonyl, acetyl, phenacylsulfonyl, trifluoromethanesulfonyl and bisbenzenesulfonyl groups etc. Splitting the protective group off can be easily achieved by conventional hydrolysis using acids or bases.

The reactions (b) can be advantageously carried out by reacting the compounds (II) and (III) in equimolar amounts or with excess of (III) in an organic solvent inert to the reaction such as methanol, ethanol, toluene, tetrahydrofuran, etc., under cooling or at room temperature. Taking various reaction conditions into account, the reaction time can be appropriately set.

In order to produce asymmetric amines, the compounds (IV) are then reacted with the secondary amines obtained in the reaction described above.

The reaction conditions and procedures are similar to those described above.

**Process 2:**

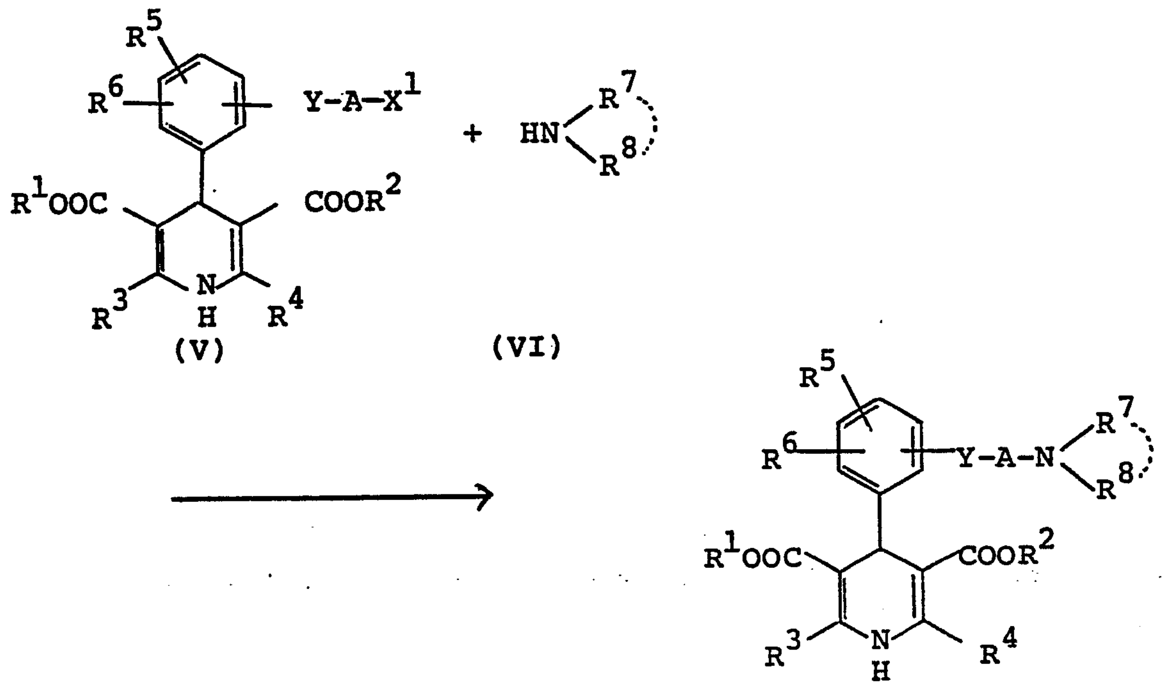
(V) (VI)

(I)

wherein $R^1$, $R^2$, $R^3$, $R^4$, $R^5$, $R^6$, $R^7$, $R^8$, Y, A and $X^1$ are as defined above.

Compounds (I) of the present invention can thus be produced by reacting halides or sulfonates ( V) with amines (VI).

The reaction can be similar to the N-alkylation in Process 1.

**Process 3:**

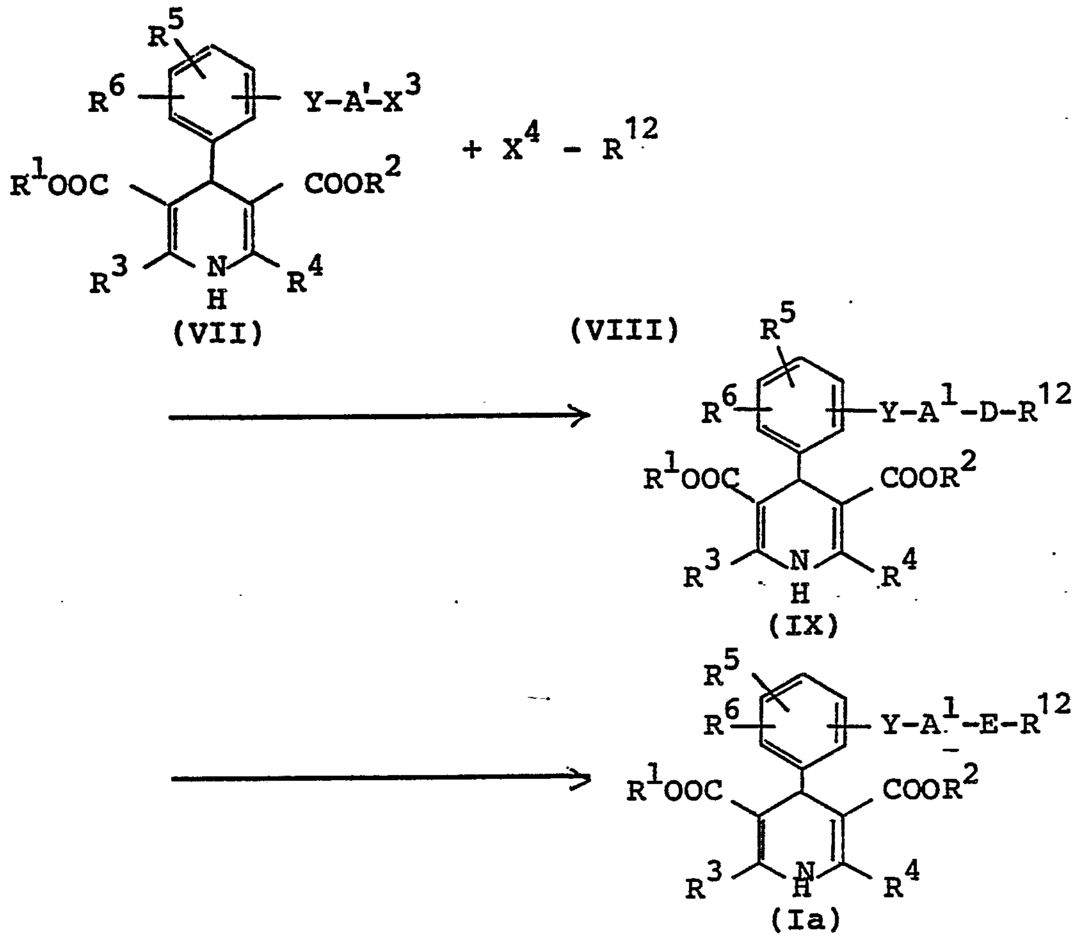

(VII)

(VIII)

(IX)

(Ia)

wherein $R^1$, $R^2$, $R^3$, $R^4$, $R^5$, and $R^6$, are as defined above;
one of $X^3$ and $X^4$ is formyl and the other amino;
D is -CH=N-or -N=CH-when $X^3$ or $X^4$ respectively is formyl; and
E is $-CH_2NH$-or $-NHCH_2$-when $X^3$ or $X^4$ respectively is formyl; and
when $X^3$ is formyl $A'-CH_2$-is A and $R^{12}$ is $R^8$ and
when $X^4$ is formyl A' is A and $-CH_2-R^{12}$ is $R^8$.

Compounds represented by general formula(Ia) and thus produced by reacting aldehydes or amines (VII) with amines or aldehydes (VIII) with reductive condensation of the resulting Schiff's bases (IX) under conditions such that any nitro group is not reduced.

The first stage reaction can proceed in the absence of any solvent but generally uses equimolar amounts of compounds (VII) and (VIII) or an excess of either compound at room temperature or with heating or with heating under reflux in an organic solvent inert to the reaction such as alcohols, e.g., methanol, ethanol or the like, or benzene, etc. Depending upon the reaction conditions, it may sometimes be advantageous to incorporate potassium hydroxide or remove the released water by a Dean-Stark trap.

For the reduction, the reducing agent can be added to the reaction solution containing the Schiff's bases without isolating the latter.

In order to produce the compounds (Ia) of the present invention by selectively reducing the imino group in the Schiff's bases, without reducing the nitro group, it is advantageous to use as the reducing agent a borohydride such as sodium borohydride, lithium borohydride, sodium cyanoborohydride, etc.

The reduction can be carried out in an organic solvent such as an alcohol (e.g., methanol, ethanol or the like) or acetic acid, or water, or a solvent mixture thereof, generally at room temperature or with heating.

For smooth operation it is sometimes advantageous to maintain the reaction solution at a neutral or basic pH; e.g. by adding methylamine, ethylamine, propylamine, dimethylamine, potassium hydroxide, sodium acetate, or the like.

Other Processes

The above processes involve reaction of a substituted alkoxy or alkylthio group on the required skeleton with the appropriate reactants to convert it to the required

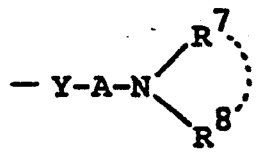

substituent. The compounds of the present invention can also be produced by synthesis of other required functional groups on an appropriate skeleton, or by synthesis of the 1,4-dihydropyridine skeleton (Hantzsch's synthesis method).

The compounds of the present invention can be used as they are, in a free state, or as salts thereof. Isolation and purification can be performed by conventional chemical operations such as extraction, crystallization, recrystallization, various chromatography techniques, etc.

In the compounds of the present invention, racemates, optically active forms, diastereoisomers and the like may be present singly or in admixture. Stereochemically pure isomers can be obtained using appropriate raw compounds or by conventional racemic resolution [for example, by treatment with conventional optically active acid (tartaric acid, etc.) followed by optical resolution, etc.]. A mixture of diastereoisomers can be separated in conventional manner, for example, fractional crystallization, chromatography, etc.

The compounds (I) and salts thereof provided by the present invention possess a calcium antagonistic action having excellent durability.

The pharmacological effects of compounds of the present invention have been confirmed by the test methods described later. With respect to hypotensive effect and coronary blood flow increasing effect, the compounds are effective in a range of 0.01 to 1 mg/kg by intravenous administration and with respect to coronary vasodilating action by administration in the coronary artery, in a range of 1 to 300 µg. In addition, reduced working load on the heart and decrease in oxygen consumption of the heart muscle were noted. It has also been confirmed that the duration of the pharmacological effects is longer than for known dihydropyridine compounds.

Accordingly the compounds of the present invention show vasodilatory activity in the body blood vessels and the coronary artery based on the calcium antagonistic action and are useful as agents for prophylaxis and treatment of ischemic heart disorders such as angina pectoris, myocardial infarction, etc. as well as circulatory diseases such as hypertension, arrhythmia, etc.; side effects may be reduced as compared to known dihydropyridine calcium antagonists and the new compounds can be applicable in less administration time. Further, the compounds of the present invention can act to improve cerebro-vascular contraction and to improve central functions.

Compositions containing one or more compounds (I) or salts thereof as effective components can be prepared as tablets, powders, granules, granulates, capsules, pills, liquid, injections, suppositories, ointments, haps, etc. using conventional carriers, excipients, etc., and other additives, and can be administered orally (including sublingually) or parenterally.

The carriers and excipients for medical preparations include solid or liquid non-toxic pharmaceutical substances. Examples of such substances are lactose, magnesium stearate, starch, talc, gelatin, agar, pectin, gum arabic, olive oil, sesame oil, cacao butter, ethylene glycol, etc. and other substances ordinarily used.

Clinical dosage of the compounds of the prsent invention may be appropriately determined depending upon condition, body weight, age, sex, etc. of the patient to be treated, but is generally 50 to 200 mg daily for an adult, administered in one dose or two sub-doses.

Test methods used for assessing compounds according to the invention were as follows:

1. $Ca^{2+}$-Antagonistic activity:

$Ca^{2+}$-Antagonistic activity of the compound was evaluated in [$^3$H]-nitrendipine ([$^3$H]-NIT) binding assay. The inhibitory effect of the test compound on [$^3$H]-NIT binding to rat brain membranes was evaluated according to the method of Gould, Murphy and Snyder ([$^3$H]-Nitrendipine-labeled calcium channels discriminate inorganic calcium agonists and antagonists. (Proc. Nati. Acad, Sci. USA., 79, 3656-3660, 1982). The $IC_{50}$ values, the concentration required to inhibit specific binding by 50%, were computed by logit-log analysis and then the inhibition constant (Ki value in nM) was obtained (Table 1).

Table 1

Effect on [$^3$H]-nitrendipine binding in rat brain membrane preparations.

| Compound | Ki (nM) | Hill coefficient |
|---|---|---|
| Example 1 | 75 (60-95) | 1.04 |
| Nifedipine | 4.6 (4.2-5.2) | 1.10 |

Value are the mean of 3 experiments.

Figures in parenthesis represent 95% confidence limits.

2. Hypotensive activity:

The effect of the compound on arterial blood pressure (systolic, SBP mean, MPB diastolic, DBP) and heart rate (HR) was evaluated in conscious normotensive rats (NTR). Four to six days before the experiments, a polyethylene catheter for recording arterial blood pressure was surgically implanted into the thoracic aorta via the carotid artery under ether anesthesia. Arterial blood pressure and heart rate were recorded under freely moving conditions. The test compounds were suspended in 0.5% methylcellulose solution and administered by oral gavage.

Table 2 summarizes the changes in HR, SBP, MBP and DBP of conscious NTR after oral administration of the test compounds.

Table 2-a: Effect of oral administration of the Example 1 compound (30 mg/kg) on heart rate (HR), systolic blood pressure (SBP), mean blood pressure (MBP) and diastolic blood pressure (DBP) in cannulated conscious normotensive rats. Value are the mean ± S.E.M. of 6 rats.

30 mg/kg p.o. (n=6)
the Example 1 compound

| Parameter | Initial Value | Change (Δ) Time after oral administration (hr) 1 | 2 | 3 | 4 | 5 | 6 | 8 | 10 | 24 |
|---|---|---|---|---|---|---|---|---|---|---|
| HR (beats/min) | 361 ±7.7 | 43.0** ±9.9 | 77.8** ±11.7 | 62.2** ±11.6 | 55.0** ±10.7 | 55.3** ±12.6 | 30.5* ±11.6 | 33.0 ±14.1 | 22.5 ±14.1 | 28.0 ±19.1 |
| SBP (mmHg) | 137 ±3.7 | −10.2 ±4.3 | −17.2* ±6.5 | −24.3* ±6.7 | −18.7** ±4.6 | −16.2* ±4.4 | −16.8* ±5.0 | −15.3** ±2.9 | −14.7** ±2.1 | 0.7 2.1 |
| MBP (mmHg) | 114 ±3.6 | −12.1* ±4.1 | −20.0* ±5.4 | −25.1** ±5.2 | −18.9** ±3.6 | −16.5** ±3.3 | −14.4* ±4.1 | −13.5** ±2.1 | −13.1** ±1.9 | 0.7 2.2 |
| DBP (mmHg) | 103 ±3.6 | −13.0* ±4.0 | −21.3** ±4.9 | −25.5** ±4.4 | −19.0** ±3.3 | −16.7** ±3.0 | −13.2* ±3.7 | −12.5** ±1.8 | −12.3** ±2.1 | 0.7 2.3 |

Significantly different from initial value (* $p < 0.05$, ** $p < 0.01$ paired t-test).

Table 2-b: Effect of oral administration of nifedipine (3 mg/kg) on heart rate (HR), systolic blood pressure (SBP), mean blood pressure (MBP) and diastolic blood pressure (DBP) in cannulated conscious mormotensive rats. Value are the mean ± S.E.M. of 8 rats.

Nifedipine 3 mg/kg p.o. (n=8)

| Parameter | Initial Value | Change (Δ) Time after oral administration (hr) 0.25 | 0.5 | 0.75 | 1 | 1.5 | 2 | 3 | 4 |
|---|---|---|---|---|---|---|---|---|---|
| HR (beats/min) | 349 ±6.9 | 77.4** ±11.9 | 51.6** ±11.2 | 35.4* ±14.8 | 23.0 ±18.3 | 12.9 ±14.4 | 2.3 ±16.7 | -20.3 ±11.4 | -13.4 ±13.7 |
| SBP (mmHg) | 136 ±2.2 | -14.4* ±4.4 | -17.6** ±4.4 | -18.1** ±3.9 | -17.0** ±2.7 | -11.0** ±2.9 | -10.4* ±3.1 | -5.4 ±4.5 | -0.3 ±3.6 |
| MBP (mmHg) | 110 ±1.8 | -18.7** ±3.5 | -19.1** ±2.9 | -19.5** ±2.1 | -18.3** ±1.6 | -11.5** ±2.9 | -9.7* ±2.9 | -3.5 ±4.4 | 0.5 ±3.5 |
| DBP (mmHg) | 97 ±1.8 | -20.9** ±3.2 | -19.9** ±2.5 | -20.1** ±1.9 | -18.9** ±1.6 | -11.8** ±3.0 | -9.4* ±3.0 | -2.6 ±4.4 | 0.9 ±3.6 |

Significantly different from initial value (* $p < 0.05$, ** $p < 0.01$ paired t-test).

3. Acute toxicity:

Acute toxicity of the test compound was evaluated according to the method of Brownlee, Hodges and Rosenblatt (The Up and Down Method with Small Samples. J. Am. Stat. As., 48, 262-277, 1953). The $ID_{50}$ value of the test compound after intravenous injection into ICR-SLC mice was obtained (Table 3).

Table 3

Acute toxicity in mice

| Compound | n | $LD_{50}$ (mg/kg i.v.) |
|---|---|---|
| Example 1 | 10 | 29.0 |
| Verapamil | 10 | 10.3 |

Example 1

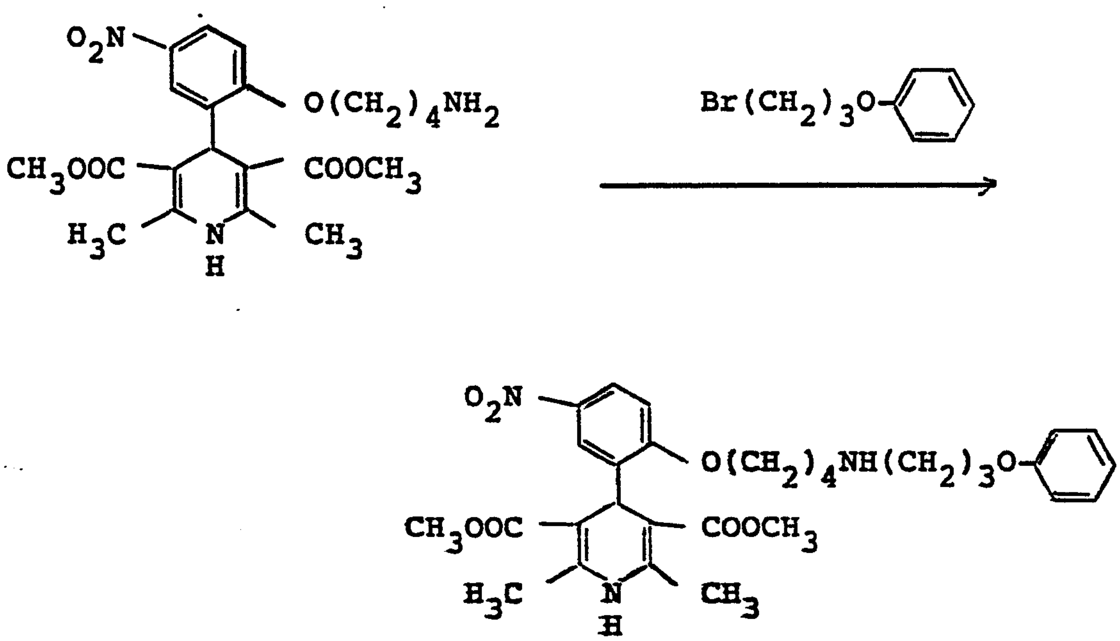

In 20 ml of N,N-dimethylformamide was dissolved 1 g of dimethyl 4-[2-(4-aminobutoxy)-5-nitrophenyl]-2,6-dimethyl-1,4-dihydropyridine-3,5-dicarboxylate. The solution was heated at 100°C for 1.5 hours. The reaction solution was carefully poured onto 500 ml of ice water. The precipitated oily substance was extracted with ethyl acetate. After drying over anhydrous sodium sulfate, the solvent was removed by distillation under reduced pressure. The obtained residue was subjected to silica gel column chromatography and eluted by chloroform : methanol (96 : 4 v/v). The obtained crude crystals were recrystallized from ethanol to give 1.2 g of dimethyl 4-[2-[4-(3-phenoxypropylamino)butoxy]-5-nitrophenyl]-2,6-dimethyl-1,4-dihydropyridine-3,5-dicarboxylate. This compound has the following physicochemical properties.

(i) Melting point: 128 - 130°C

(ii) Elemental analysis (as $C_{30}H_{37}N_3O_8 \cdot H_2O$)

| | C(%) | H(%) | N(%) |
|---|---|---|---|
| Calcd. | 61.53 | 6.71 | 7.17 |
| Found | 61.42 | 6.45 | 7.00 |

Example 2

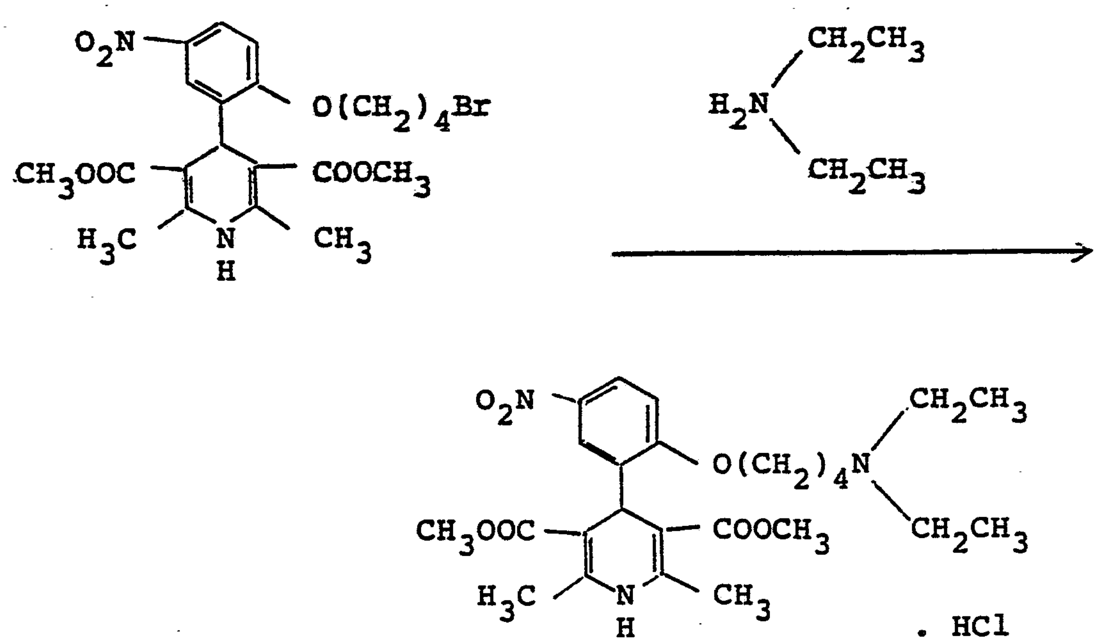

In 30 ml of acetonitrile were dissolved 3 g of dimethyl 4-[2-(4-bromobutoxy)-5-nitrophenyl]-2,6-dimethyl-1,4-dihydropyridine-3,6-dicarboxylate and 0.88 g of diethylamine. The solution was heated to reflux for 2.5 hours. The reaction solution was carefully poured onto 500 ml of ice water. The solvent was removed by distillation under reduced pressure. The obtained residue was dissolved in methylene chloride followed by washing with 10% sodium hydroxide aqueous solution and then with 1N hydrochloride. The solvent was removed from the organic phase by distillation under reduced pressure. The obtained crude crystals were recrystallized from methanol-ethanol to give 1.3 g of dimethyl 4-[2-(4-diethylaminobutoxy]-5-nitrophenyl]-2,6-dimethyl-1,4-dihydropyridine-3,5-dicarboxylate hydrochloride. This compound has the following physicochemical properties.

(i) Melting point: 216 - 219°C

(ii) Elemental analysis (as $C_{25}H_{36}N_3O_7Cl \cdot 0.3H_2O$)

| | C(%) | H(%) | N(%) | Cl(%) |
|---|---|---|---|---|
| Calcd. | 56.50 | 6.94 | 7.91 | 6.67 |
| Found | 56.49 | 6.84 | 7.71 | 6.40 |

(iii) Nuclear magnetic resonance (DMSO-$d_6$)

δ : 1.26 (6H, t) 1.7-2.0 (4H, m)

2.29 (6H, s)　　3.52 (6H, s)

4.0-4.2 (2H, m)　　5.21 (1H, s)

7.12 (1H, d)　　7.9-8.1 (2H, m)

9.21 (1H, s)

Examples 3 to 13

The following compounds were obtained in a similar manner.

Example 3

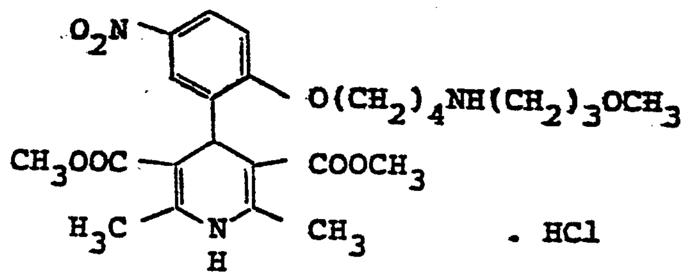

Dimethyl 4-[2-(4-butylaminobutoxy)-5-nitrophenyl]-2,6-dimethyl-1,4-dihydropyridine-3,5-dicarboxylate hydrochloride

Physicochemical properties

(i) Melting point: 220 - 222°C

(ii) Elemental analysis (as $C_{25}H_{36}N_3O_7Cl$)

| | C(%) | H(%) | N(%) | Cl(%) |
|---|---|---|---|---|
| Calcd. | 57.08 | 6.90 | 7.99 | 6.74 |
| Found | 56.79 | 6.80 | 7.86 | 6.49 |

(iii) Nuclear magnetic resonance (DMSO-$d_6$)

δ : 0.91 (3H, t)　　5.20 (1H, s)

2.28 (6H, s)　　7.13 (1H, d)

2.8-3.1 (4H, m)　　7.95-8.10 (2H, m)

3.5 (6H, s)　　9.26 (1H, s)

4.0-4.2 (2H, m)

Example 4

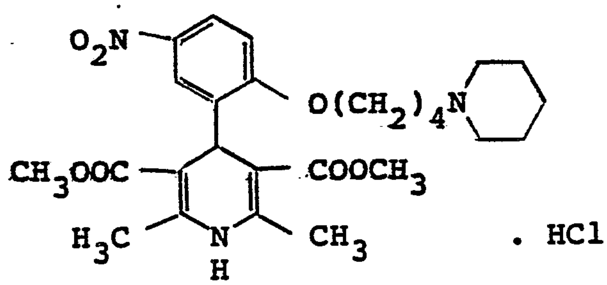

Dimethyl 4-[2-[4-(1-piperidino)butoxy]-5-nitrophenyl]-2,6-dimethyl-1,4-dihydropyridine-3,5-dicarboxylate hydrochloride

Physicochemical properties

(i) Melting point: 214 - 218°C

(ii) Elemental analysis (as $C_{26}H_{36}N_3O_7Cl.0.3H_2O$)

| | C(%) | H(%) | N(%) | Cl(%) |
|---|---|---|---|---|
| Calcd. | 57.46 | 6.79 | 7.73 | 6.52 |
| Found | 57.44 | 6.70 | 7.71 | 6.65 |

(iii) Nuclear magnetic resonance (DMSO-$d_6$)

δ : 1.6-2.0 (8H, m) 5.19 (1H, s)
2.24 (6H, s) 7.09 (1H, d)
3.47 (6H, s) 7.9-8.1 (2H, m)
4.0-4.2 (2H, m) 9.17 (1H, s)

Example 5

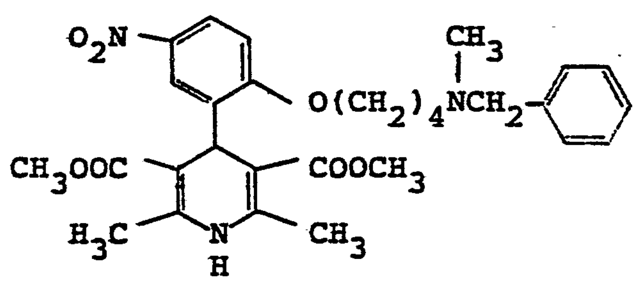

Dimethyl 4-[2-[4-(N-benzyl-N-methylaminobutoxy)-5-nitrophenyl]-2,6-dimethyl-1,4-dihydropyridine-3,5-dicarboxylate

Physicochemical properties

(i) Melting point: 73 - 74°C

(ii) Elemental analysis (as $C_{29}H_{35}N_3O_7$)

| | C(%) | H(%) | N(%) |
|---|---|---|---|
| Calcd. | 64.79 | 6.56 | 7.82 |
| Found | 64.63 | 6.69 | 7.83 |

(iii) Nuclear magnetic resonance ($CDCl_3$)

δ : 1.5-2.1 (4H, m) 3.58 (6H, s)
2.26 (3H, s) 4.04 (2H, t)
2.30 (6H, s) 5.34 (1H, s)
2.46 (2H, t) 6.82 (1H, d)
3.52 (2H, s) 7.2-7.4 (5H, m)
7.9-8.2 (2H, m)

Example 6

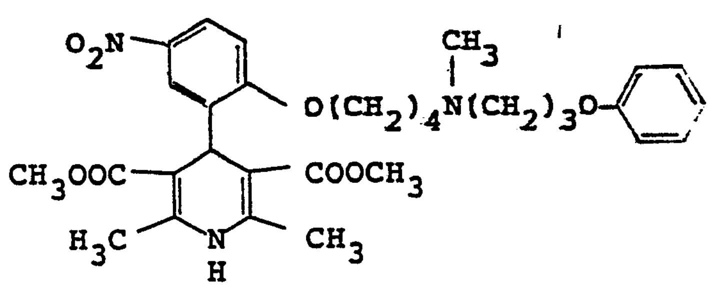

Dimethyl 4-[5-nitro-2-[4-[N-methyl-N-(3-phenoxypropyl)amino]butoxy]phenyl]-2,6-dimethyl-1,4-dihydropyridine-3,5-dicarboxylate

Physicochemical properties

(i) Melting point: 176 - 179°C

(ii) Elemental analysis (as $C_{31}H_{39}N_3O_8$)

| | C(%) | H(%) | N(%) |
|---|---|---|---|
| Calcd. | 64.01 | 6.76 | 7.22 |
| Found | 63.89 | 6.64 | 7.32 |

(iii) Nuclear magnetic resonance ($CDCl_3$)

δ : 1.8-2.4 (6H, m) 3.9-4.2 (4H, m)

2.34 (6H, s) 5.32 (1H, s)

2.52 (3H, s) 6.7-7.1 (4H, m)

2.7-3.0 (4H, m) 7.2-7.4 (2H, m)

3.58 (2H, s) 7.9-8.2 (2H, m)

Example 7

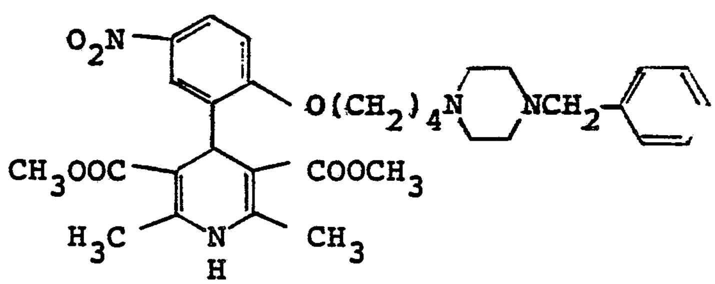

Dimethyl 4-[2-[4-(4-benzyl-1-piperazinyl)butoxy)-5-nitrophenyl]-2,6-dimethyl-1,4-dihydropyridine-3,5-dicarboxylate

Physicochemical properties

(i) Melting point: 81 - 83°C

(ii) Elemental analysis (as $C_{32}H_{40}N_4O_7$)

| | C(%) | H(%) | N(%) |
|---|---|---|---|
| Calcd. | 64.85 | 6.80 | 9.45 |
| Found | 64.41 | 6.72 | 9.49 |

(iii) Nuclear magnetic resonance ($CDCl_3$)

δ : 1.4-2.1 (8H, m) 4.02 (2H, t)

2.28 (6H, s) 5.26 (1H, s)

2.6-2.9 (2H, m) 6.82 (1H, d)

3.52 (2H, s) 7.2-7.4 (5H, m)

3.56 (6H, s) 7.9-8.2 (2H, m)

Example 8

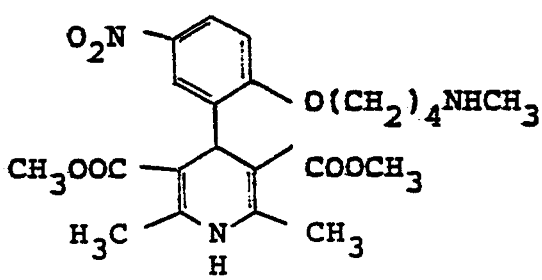

Dimethyl 4-[2-[4-(N-methylamino)butoxy]-5-nitrophenyl]-2,6-dimethyl-1,4-dihydropyridine-3,5-dicarboxylate

Physicochemical properties

(i) Melting point: 167 - 169°C

(ii) Elemental analysis (as $C_{22}H_{29}N_3O_7$)

| | C(%) | H(%) | N(%) |
|---|---|---|---|
| Calcd. | 59.05 | 6.53 | 9.39 |
| Found | 58.78 | 6.62 | 9.28 |

(iii) Nuclear magnetic resonance ($CDCl_3$)

| | | |
|---|---|---|
| δ : | 1.5-2.1 (4H, m) | 4.04 (2H, t) |
| | 2.30 (6H, s) | 5.28 (1H, s) |
| | 2.48 (3H, s) | 6.82 (1H, d) |
| | 2.72 (2H, t) | 7.9-8.2 (2H, m) |
| | 3.56 (6H, s) | |

Example 9

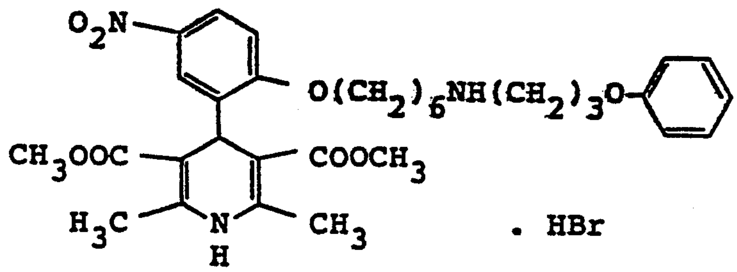

. HBr

Dimethyl 4-[2-[4-(3-phenoxypropylamino)hexyloxy]-5-nitrophenyl]-2,6-dimethyl-1,4-dihydropyridine-3,5-dicarboxylate hydrobromide

Physicochemical properties

(i) Melting point: 197 - 198°C

(ii) Elemental analysis (as $C_{32}H_{42}N_3O_8Br$)

| | C(%) | H(%) | N(%) |
|---|---|---|---|
| Calcd. | 56.81 | 6.26 | 6.21 |
| Found | 57.20 | 6.25 | 6.33 |

(iii) Nuclear magnetic resonance (DMSO-$d_6$)

δ : 1.3-2.3 (8H, m) 2.28 (6H, s)

2.8-3.4 (4H, m) 6.50 (6H, s)

4.0-4.2 (4H, m) 5.22 (1H, s)

6.8-7.4 (6H, m) 7.9-8.1 (2H, m)

Example 10

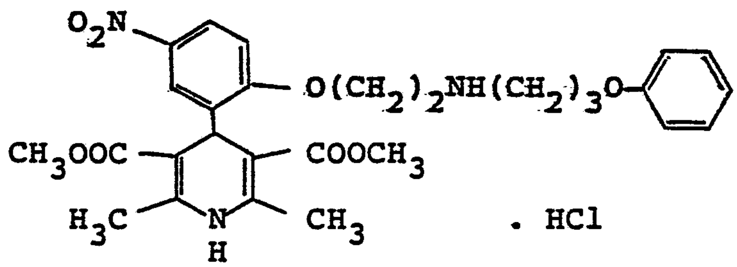

. HCl

Dimethyl 4-[2-[4-(3-phenoxypropylamino)ethoxy]-5-nitrophenyl]-2,6-dimethyl-1,4-dihydropyridine-3,5-dicarboxylate hydrochloride

Physicochemical properties

(i) Melting point: 118 - 120°C

(ii) Elemental analysis (as $C_{28}H_{34}N_3O_8Cl$)

| | C(%) | H(%) | N(%) | Cl(%) |
|---|---|---|---|---|
| Calcd. | 58.38 | 5.95 | 7.29 | 6.15 |
| Found | 58.55 | 5.87 | 7.17 | 6.54 |

(iii) Nuclear magnetic resonance (DMSO-$d_6$)

δ : 2.28 (6H, s) 3.2-3.6 (12H, m)
4.12 (2H, t) 4.50 (2H, t)
5.26 (1H, s) 6.8-7.5 (6H, m)
8.0-8.2 (2H, m)

Example 11

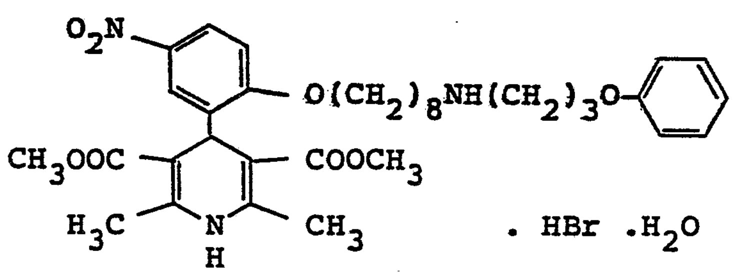

. HBr .$H_2O$

Dimethyl 4-[2-[4-(3-phenoxypropylamino)octyloxy]-5-nitrophenyl]-2,6-dimethyl-1,4-dihydropyridine-3,5-dicarboxylate hydrobromide monohydrate

Physicochemical properties

(i) Amorphous powders

(ii) Elemental analysis (as $C_{34}H_{48}N_3O_9Br$)

| | C(%) | H(%) | N(%) |
|---|---|---|---|
| Calcd. | 60.66 | 6.96 | 6.24 |
| Found | 60.68 | 6.96 | 6.26 |

(iii) Nuclear magnetic resonance (DMSO-$d_6$)

δ : 1.2-1.9 (8H, m) 2.0-2.4 (6H, m)
2.7-3.2 (4H, m) 3.52 (6H, s)
4.08 (4H, t) 5.24 (1H, s)
6.9-7.4 (6H, m) 7.9-8.1 (2H, m)
8.04 (1H, s)

Example 12

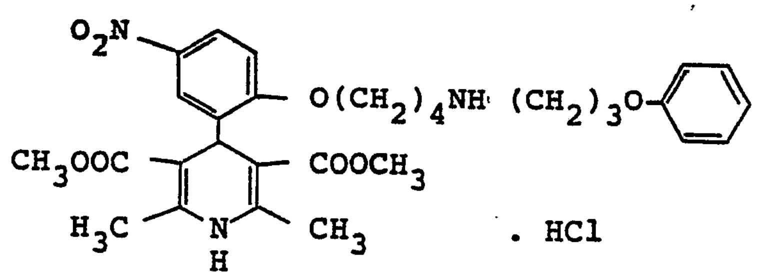

Dimethyl 4-[2-[4-(3-phenoxypropylamino)butoxy]-5-nitrophenyl]-2,6-dimethyl-1,4-dihydropyridine-3,5-dicarboxylate hydrochloride

Physicochemical properties

(i) Melting point: 155 - 158°C

(ii) Elemental analysis (as $C_{30}H_{38}N_3O_9Cl$)

| | C(%) | H(%) | N(%) | Cl(%) |
|---|---|---|---|---|
| Calcd. | 58.11 | 6.18 | 6.78 | 5.72 |
| Found | 57.92 | 6.12 | 6.79 | 5.45 |

(iii) Nuclear magnetic resonance (DMSO-$d_6$)

δ : 1.6-2.3 (6H, m) 2.28 (6H, s)
3.3-3.6 (2H, m) 3.50 (6H, s)
3.9-4.2 (6H, m) 5.20 (1H, s)
6.8-7.4 (6H, m) 7.9-8.2 (2H, m)
9.08 (1H, s)

Example 13

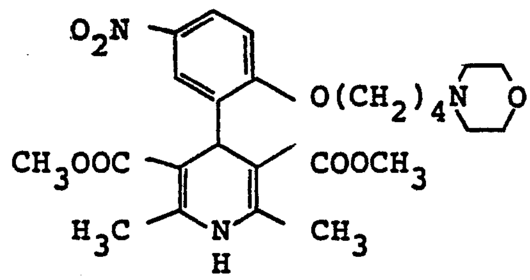

Dimethyl 4-[2-[4-(morpholino)butoxy]-5-nitrophenyl]-2,6-dimethyl-1,4-dihydropyridine-3,5-dicarboxylate

Physicochemical properties

(i) Melting point: 195 - 197°C

(ii) Elemental analysis (as $C_{25}H_{33}N_3O_8$)

| | C(%) | H(%) | N(%) |
|---|---|---|---|
| Calcd. | 59.63 | 6.61 | 8.34 |
| Found | 59.45 | 6.60 | 8.00 |

(iii) Nuclear magnetic resonance ($CDCl_3$)

δ : 1.4–2.0 (4H, m) 2.0–2.6 (10H, m)
3.3–3.8 (10H, m) 4.12 (2H, t)
5.24 (1H, s) 7.10 (1H, d)
7.9–8.1 (2H, m)

Example 14

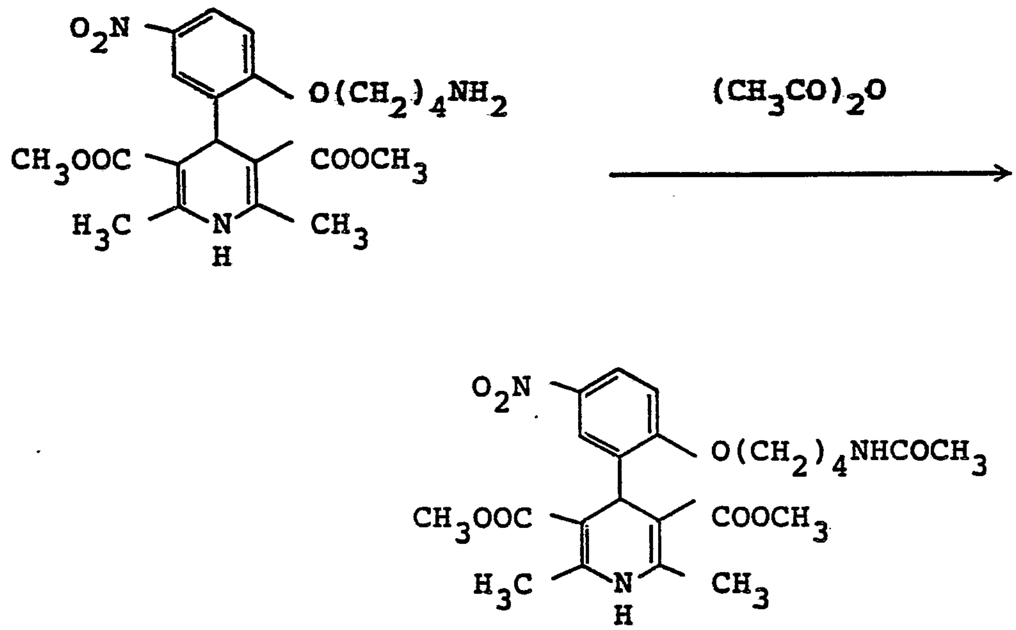


In 2 ml of methylene chloride was dissolved 0.31 g of dimethyl 4-[2-(4-aminobutoxy)-5-nitrophenyl]-2,6-dimethyl-1,4-dihydropyridine-3,5-dicarboxylate. After 2 ml of acetic anhydride was added to the solution under ice cooling, the mixture was stirred for 30 minutes at room temperature. The reaction solution was concentrated under reduced pressure and ether was added to the residue to solidify. The solid was recrystallized from chloroform-n-hexane to give 0.26 g of dimethyl 4-[2-[4-(acetylamino)butoxy]-5-nitrophenyl]-2,6-dimethyl-1,4-dihydropyridine-3,5-dicarboxylate. This compound has the following physicochemical properties.

(i) Melting point: 212 - 213°C

(ii) Elemental analysis (as $C_{23}H_{29}N_3O_8$)

| | C(%) | H(%) | N(%) |
|---|---|---|---|
| Calcd. | 58.10 | 6.15 | 8.84 |
| Found | 57.67 | 6.01 | 8.74 |

Example 15

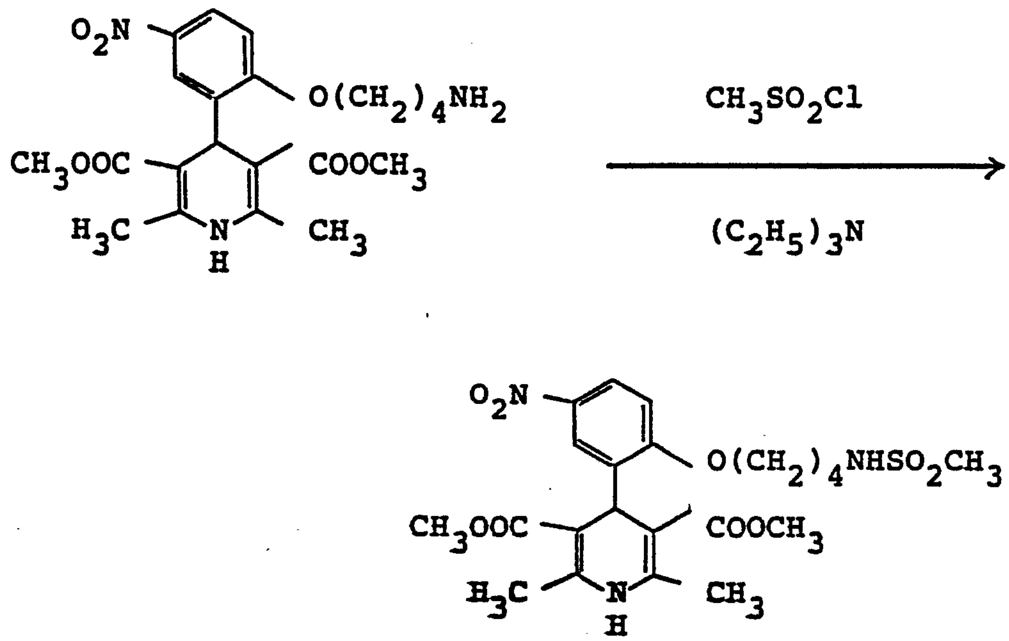


In 4 ml of methylene chloride was dissolved 0.43 g of dimethyl 4-[2-(4-aminobutoxy)-5-nitrophenyl]-2,6-dimethyl-1,4-dihydropyridine-3,5-dicarboxylate. After 0.138 ml of triethylamine and 0.077 ml of methanesulfonyl chloride were added to the solution under ice cooling the mixture was stirred for 3 days at room temperature. To the reaction solution were added 50 ml of ethyl acetate and 20 ml of 2N HCl to fractionate the organic phase. The solvent was removed by distillation under reduced pressure. The obtained residue was subjected to silica gel column chromato graphy and eluted by chloroform : methanol (9 : 1 v/v). The obtained crude crystals were recrystallized from chloroform-n-hexane to give 0.31 g of dimethyl 4-[2-[4-(methanesulfonylamino)butoxy]-5-nitrophenyl]-2,6-dimethyl-1,4-dihydropyridine-3,5-dicarboxylate. This compound has the following physicochemical properties.

(i) Melting point: 213 - 216°C

(ii) Elemental analysis (as $C_{22}H_{29}N_3O_9S \cdot 1/2H_2O$)

| | C(%) | H(%) | N(%) |
|---|---|---|---|
| Calcd. | 50.76 | 5.81 | 8.07 |
| Found | 50.09 | 5.44 | 7.83 |

Reference Example 1

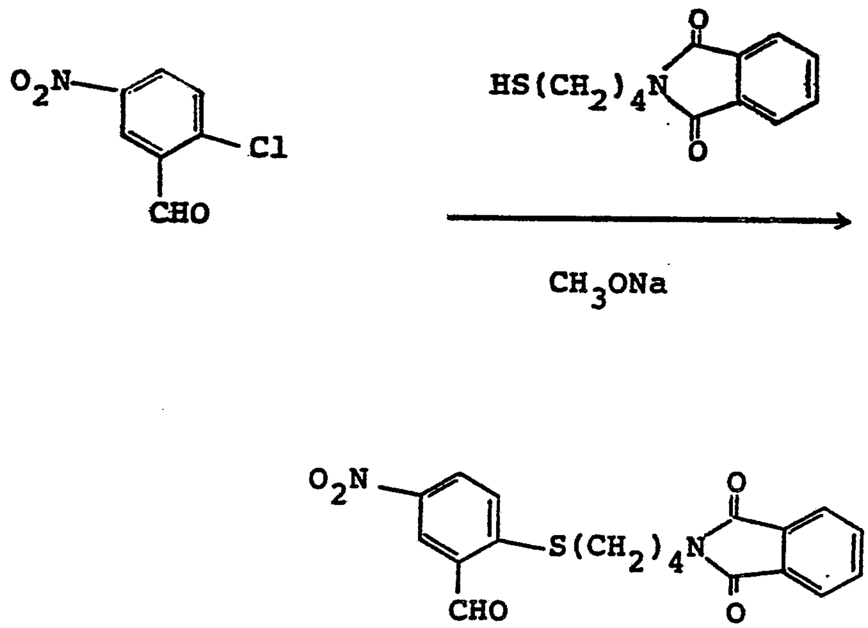


In 40 ml of methanol was dissolved 0.7 g of metallic sodium. A solution of 7.16 g of 4-phthalimidobutylthiol was added to the solution followed by stirring at room temperature for 15 minutes. Then, 4.64 g of 2-chloro-5-nitrobenzaldehyde was added to the reaction mixture, which was heated to reflux for 6 hours. After the solvent was removed by distillation under reduced pressure, 15 ml of methanol was added to the residue and the precipitates were taken out by filtration. The precipitates were washed with water and methanol to give 3.94 g of 5-nitro-2-(4-phthalimidobutylthio)benzaldehyde.

Nuclear magnetic resonance (DMSO-$d_6$)

| | | |
|---|---|---|
| δ : | 1.5-2.0 (4H, m) | 3.16 (2H, t) |
| | 3.64 (2H, t) | 7.70 (1H, d) |
| | 7.86 (4H, bs) | 8.32 (1H, d) |
| | 8.70 (1H, d) | 10.16 (1H, s) |

Reference Example 2

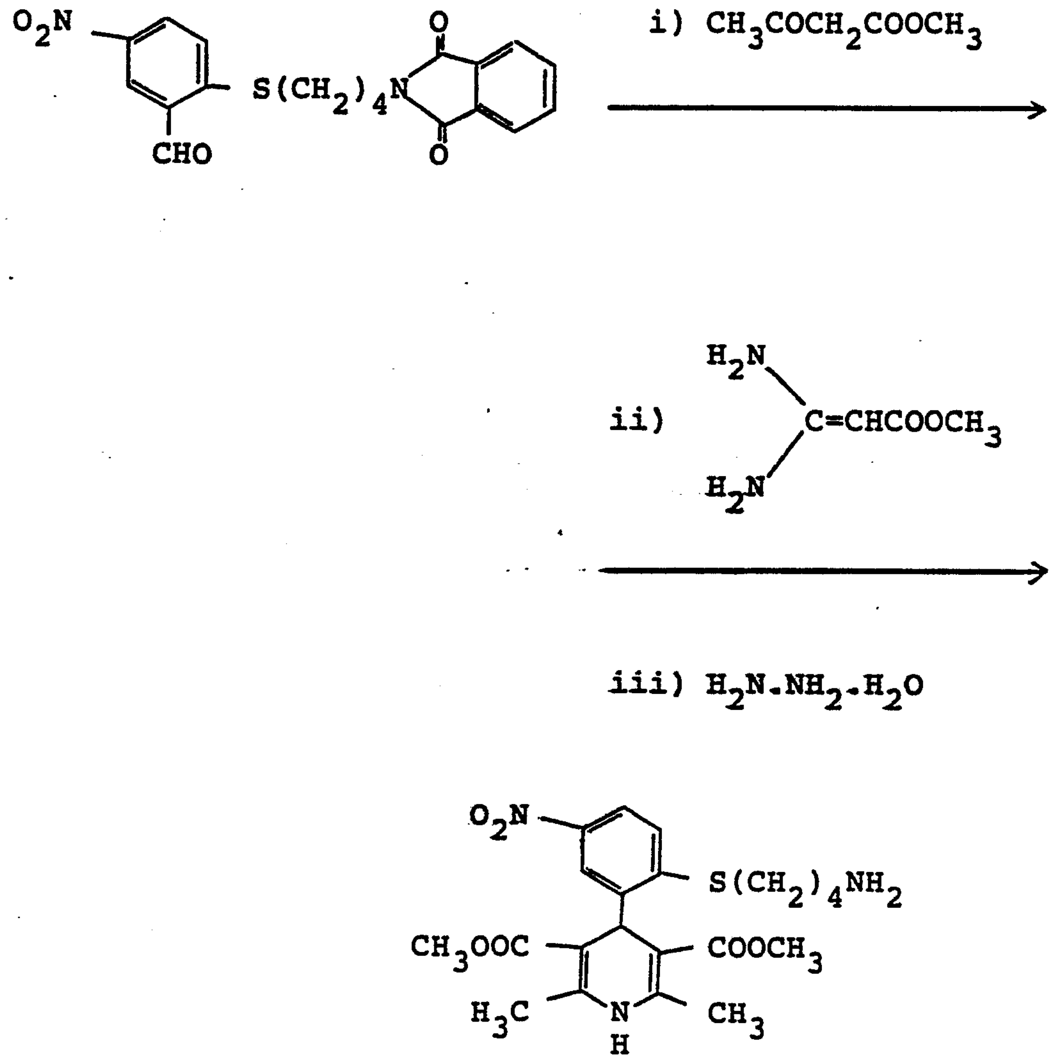


1) To 4 g of 5-nitro-2-(4-phthalimidobutylthio)benzaldehyde were added 100 ml of benzene, 1.21 g of methyl acetoacetate, 0.04 ml of piperidine and 0.12 ml of acetic acid. The mixture was heated to reflux for 4 hours using Dean-Stark device. After cooling, the reaction mixture was washed twice with saturated sodium hydrogencarbonate aqueous solution and 2N hydrochloric acid, respectively. After drying over anhydrous sodium sulfate, the solvent was removed by distillation under reduced pressure to give an oily substance. Ether was added to the oily substance to solidify the oil. Thus, 4.9 g of the solid was obtained. The solid was used in the next step without purification.

2) To 4.9 g of the solid obtained above were added 49 ml of isopropyl alcohol and 1.17 g of methyl 3-aminocrotonate. The mixture was heated to reflux overnight. To the reaction solution were added 100 ml of ethanol and 6.38 g of hydrated hydrazine followed by heating to reflux for an hour. After cooling, insoluble matters were filtered off and the solvent was removed by distillation under reduced pressure. To the residue were added 80 ml of ethyl acetate and 80 ml of water, which was fractionated. After drying the organic phase over anhydrous sodium sulfate, the solvent was removed by distillation under reduced pressure. Ether was added to the residue to crystallize and give 3.4 g of dimethyl 4-[2-(4-aminobut ylthio)-5-nitrophenyl]-2,6-dimethyl-1,4-dihydropyridine-3,5-dicarboxylate. This compound has the following physicochemical properties.

(i) Melting point: 260 °C (decomposed)

(ii) Nuclear magnetic resonance (DMSO-$d_6$)

δ : 1.4-2.0 (4H, m) 2.24 (6H, s)

2.4-2.6 (2H, m) 2.6-2.9 (2H, m)

5.30 (1H, bs) 7.50 (1H, d)

7.90 (1H, d) 8.00 (1H, d)

Example 16

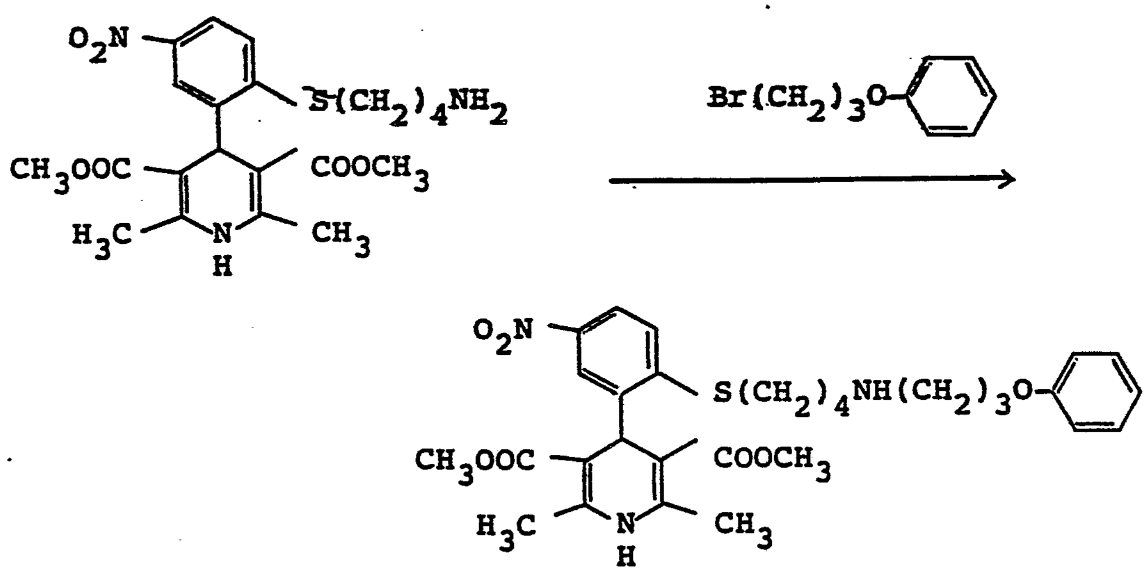

In 30 ml of acetonitrile were dissolved 1.35 g of dimethyl 4-[2-(4-aminobutylthio)-5-nitrophenyl]-2,6-dimethyl-1,4-dihydropyridine-3,5-dicarboxylate obtained in Reference Example 2 and 0.645 g of 1-bromo-3-phenoxypropane. The solution was heated to reflux for 30 mintues. The solvent was removed by distillation under reduced pressure. The obtained residue was subjected to silica gel column chromatography and eluted by chloroform : methanol (9 : 1 v/v). The obtained yellow oily substance was converted into the hydrochloride with hydrochloric acid-ethanol. By drying under reduced pressure, 0.37 g of dimethyl 4-[2-[4-(3-phenoxypropylamino)butylthio]-5-nitrophenyl]-2,6-dimethyl-1,4-dihydropyridine-3,5-dicarboxylate hydrochloride. This compound has the following physicochemical properties.

(i) Amorphous powders

(ii) Elemental analysis (as $C_{30}H_{38}N_3O_7SCl$)

| | C(%) | H(%) | N(%) |
|---|---|---|---|
| Calcd. | 58.10 | 6.18 | 6.78 |
| Found | 58.24 | 6.13 | 6.62 |

(iii) Nuclear magnetic resonance (DMSO-$d_6$)

δ : 1.6-2.0 (6H, m) 2.24 (6H, s)
2.5-3.3 (6H, m) 3.50 (6H, s)
4.60 (2H, t) 5.30 (1H, s)
7.92 (1H, d) 7.96 (1H, bs)
6.8-7.6 (6H, m) 9.12 (1H, bs)

Example 17

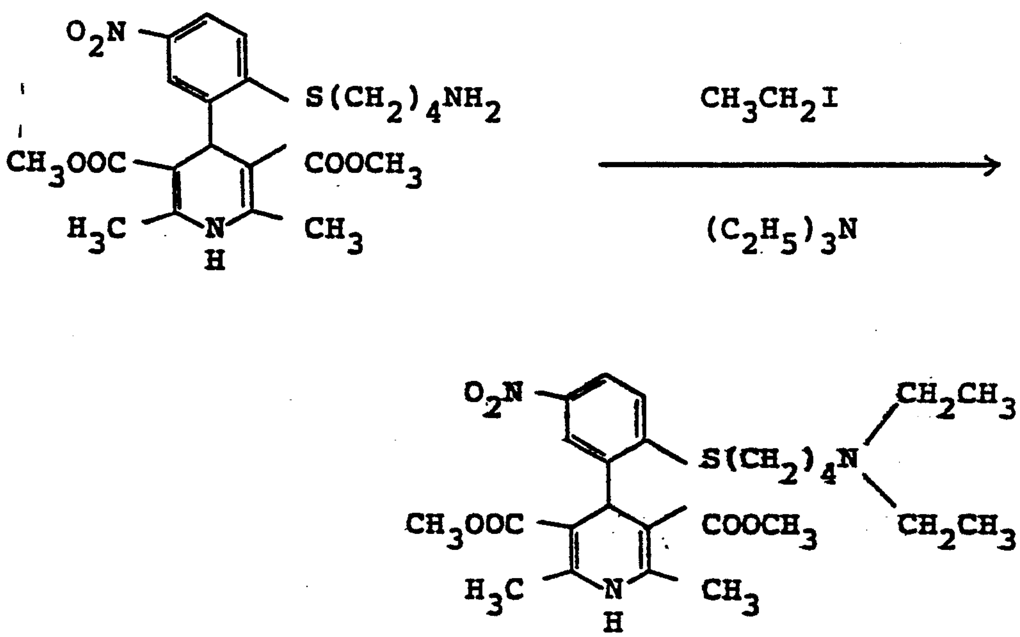


In 10 ml of acetonitrile were dissolved 0.45 g of dimethyl 4-[2-(4-aminobutylthio)-5-nitrophenyl]-2,6-dimethyl-1,4-dihydropyridine-3,5-dicarboxylate, 0.16 g of ethyl iodide and 0.10 g of triethylamine. The solution was heated to reflux for 30 minutes. The solvent was removed by distillation under reduced pressure. The obtained residue was subjected to silica gel column chromatography and eluted with ammonia-saturated chloroform : isopropyl alcohol (9 : 1 v/v). The obtained yellow oily substance was converted into the hydrochloride with hydrochloric acid-ethanol. By drying under reduced pressure, 0.19 g of dimethyl 4-[2-(4-diethylaminobutylthio)-5-nitrophenyl]-2,6-dimethyl-1,4-dihydropyridine-3,5-dicarboxylate hydrochloride. This compound has the following physicochemical properties.

(i) Amorphous powders

(ii) Elemental analysis (as $C_{25}H_{36}N_3O_6SCl$)

| | C(%) | H(%) | N(%) |
|---|---|---|---|
| Calcd. | 55.39 | 6.69 | 7.75 |
| Found | 55.74 | 6.49 | 7.69 |

(iii) Nuclear magnetic resonance (DMSO-$d_6$)

δ : 1.42 (6H, t) 1.6-2.3 (4H, m)
2.40 (6H, s) 2.8-3.4 (8H, m)
3.56 (6H, s) 5.42 (1H, bs)
7.20 (1H, d) 7.90 (1H, d)
8.20 (1H, d)

The structures of the compounds of Examples 1 to 17 may be summarised as follows :

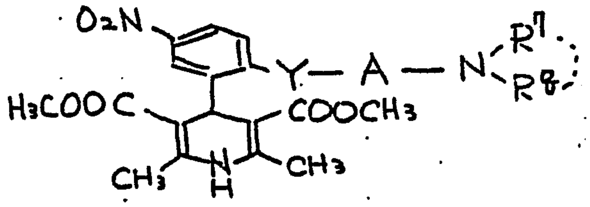

| Example No. | Y | A | $R^7$ | $R^8$ |
|---|---|---|---|---|
| 1 | -O- | $-(CH_2)_4-$ | -H | $-(CH_2)_3OPh$ |
| 2 | " | " | $-C_2H_5$ | $-C_2H_5$ |
| 3 | " | " | -H | $-C_4H_9$ |
| 4 | " | " | $-(CH_2)_5-$ | |
| 5 | " | " | $-CH_3$ | $-CH_2Ph$ |
| 6 | " | " | " | $-(CH_2)_3OPh$ |
| 7 | " | " | 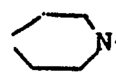$-CH_2Ph$ | |
| 8 | " | " | -H | $-CH_3$ |
| 9 | " | $-(CH_2)_6-$ | " | $-(CH_2)_3OPh$ |
| 10 | " | $-(CH_2)_2-$ | " | " |
| 11 | " | $-(CH_2)_8-$ | " | " |
| 12 | " | $-(CH_2)_4-$ | " | $-O(CH_2)_3OPh$ |
| 13 | " | " | 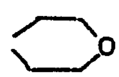 | |
| 14 | " | " | -H | $-COCH_3$ |
| 15 | " | " | " | $-SO_2CH_3$ |
| 16 | -S- | " | " | $-(CH_2)_3OPh$ |
| 17 | " | " | $-C_2H_5$ | $-C_2H_5$ |

## Claims

1. 1,4-dihydropyridine derivatives of formula (I) below, and their salts

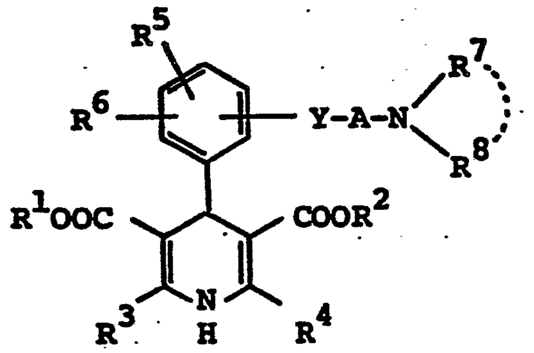

(I)

wherein
$R^1$ and $R^2$ are the same or different and selected from alkyl groups which may be interrupted at least once by an oxygen atom, cycloalkyl-substituted $C_1$-$C_6$ alkyl groups and halogen-substituted $C_1$-$C_6$ alkyl groups;
$R^3$ and $R^4$ are the same or different and selected from $C_1$-$C_6$ alkyl groups;
$R^5$ and $R^6$ are the same or different and selected from hydrogen and halogen atoms and nitro, $C_1$-$C_6$ alkyl, $C_1$-$C_6$ alkoxy, $C_1$-$C_6$ alkylthio, $C_1$-$C_6$ alkylsulfonyl and $C_1$-$C_6$ alkylsulfinyl groups;
$R^7$ is a hydrogen atom or $C_1$-$C_6$ alkyl group and
$R^8$ is a $C_1$-$C_6$ alkyl, hydroxy-($C_1$-$C_6$) alkyl, aralkyl, aryl, $C_1$-$C_6$ acyl, $C_1$-$C_6$ alkylsulfonyl, aryloxy-$C_1$-$C_6$ alkyl or aryloxy-$C_1$-$C_6$ alkoxy group or $R^7$ and $R^8$ form together with the amino nitrogen a pyrrole, piperidine, morpholine, piperazine or azepine ring which may be substituted by $C_1$-$C_6$ alkyl or aralkyl;
A is an alkylene, alkenylene or alkynylene group; and
Y is an oxygen or sulfur atom.

2. A compound according to claim 1 which is 4-[2-[4-(3-phenoxypropylamino)butoxy]-5-nitrophenyl]2,6-dimethyl-1,4-dihydropyridine-3,5-dicarboxylate or a salt thereof.

3. A process for preparing a compound according to claim 1 which comprises converting to a required

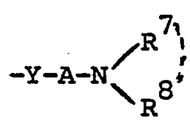

substituent a substituted alkoxy or alkylthio substituent on the phenyl group of a substituted skeleton otherwise already according to the requirements of claim.

4. A compound according to claim 1 as defined in any one of Examples 1 to 17.

5. 5-nitro-2-(4-phthalimidobutylthio)benzaldehyde or 4-[2-(4-aminobutylthio)-5-nitrophenyl]-2,6-dimethyl-1,4-dihydropyridine-3,5-dicarboxylate.

European Patent Office

# EUROPEAN SEARCH REPORT

Application Number

EP 87 31 0787

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 167 371 (YAMANOUCHI PHARMACEUTICAL CO. LTD.)<br>* abstract *<br>--- | 1 | C 07 D 211/90<br>A 61 K 31/44 |
| D,A | EP-A-0 194 751 (IMPERIAL CHEMICAL INDUSTRIES PLC)<br>* abstract *<br>--- | 1 | |
| A | EP-A-0 194 047 (IMPERIAL CHEMICAL INDUSTRIES PLC)<br>* abstract *<br>--- | 1 | |
| A | EP-A-0 194 750 (IMPERIAL CHEMICAL INDUSTRIES PLC)<br>* abstract *<br>--- | 1 | |
| A | PATENT ABSTRACTS OF JAPAN, vol. 9, no. 293 (C-315)[2016], 20th November 1985; & JP - A - 60 136 558 (TEIKOKU ZOKI SEIYAKU K.K.) 20-07-85 (Cat. D, A)<br>--- | 1 | TECHNICAL FIELDS SEARCHED (Int. Cl.4) |
| A | EP-A-0 197 488 (EISAI CO., LTD.)<br>* claim 1 *<br>----- | 1 | C 07 D 211/00 |

The present search report has been drawn up for all claims

| Place of search | Date of completion of the search | Examiner |
|---|---|---|
| BERLIN | 25-03-1988 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)